# EUROPEAN PATENT APPLICATION

(11) **EP 2 540 326 A1**
(43) Date of publication of application: **02.01.2013**
(21) Application number: 10846391.0
(22) Date of filing: 19.11.2010
(51) Int. Cl.: A61M 1/10, F04B 43/02, F04B 43/06

(54) **HYDRAULIC PULSATION EQUIPMENT FOR A PERFUSION PUMP**

(30) Priority: 25.02.2010 ES 201030263
(71) Applicant: Merce Vives, Salvador, 46004 Valencia (ES)
(72) Inventor: Merce Vives, Salvador, 46004 Valencia (ES)
(74) Representative: Ungria López, Javier
(86) International application number: PCT/ES2010/070753
(87) International publication number: WO 2011/104392

(57) **Abstract**

It is intended for varying the volume of a chamber of a perfusion pump by alternatively inflating and deflating the same by means of a movement transmitting liquid fluid. It is characterized in that it comprises a lung body (18) provided with an elastic and deformable frontal membrane (24) over which an external piston (16), which can be moved alternatively in both directions, enters into contact, and said piston originates from one of the sides of a control console (13). This console also has an enveloping ring head (17) that surrounds the external piston (16), while constituting the fixation means of the lung body (18), which has a terminal narrowing (21) connected to an intermediate tubular duct (22) culminating in the chamber (12) of the perfusion pump (23), and the movement transmitting liquid fluid is located in a space comprising a front part of the lung body (18), the intermediate tubular duct (22) and the chamber (12) of the perfusion pump (23).

## Description

### OBJECT OF THE INVENTION

The present invention, as expressed in the title of this specification, relates to a hydraulic pulsation equipment for a perfusion pump, intended to assist a blood perfusion pump of the type provided in the Spanish invention patent no. 200601000.

These types of pumps are used for blood perfusion during heart surgery or for ventricular assistance during the treatment for heart failure.

With the aforementioned pump, the perfusion system is able to provide the advantages of a lineal or a continuous flow and also a pulsatile flow, which is better for the perfusion of tissue and organs. In addition, the perfusion system may be synchronizable with the biological pulse of the patient supposing that ventricular assistance becomes necessary.

Based on this premise, the object of the invention is a typical hydraulic pulsation equipment allowing the application of a fully adjustable hydraulic pulse by means of an external piston that is a part of the equipment of the invention.

### BACKGROUND OF THE INVENTION

Nowadays, both for blood perfusion during heart surgery and for the treatment of heart failure, two types of pumps are used; roller pumps and centrifugal pumps. Both types of pumps generate a lineal or continuous flow, and therefore, a circulation of blood at a constant flow, replacing the pulsatile or physiological flow generated by the heart.

It has always been admitted that the ideal flow for perfusing tissue is the pulsatile or physiological flow because the impact produced by each heartbeat improves the perfusion of the thinnest arteries. The manufacturers of roller and centrifugal pumps have looked for solutions to transform the lineal flow into pulsatile flow, without getting satisfactory results.

Invention patent US005458459 disclosed a centrifugal pump used to pump biological liquids such as blood including a housing that defines a pumping chamber. The pumping chamber encloses a rotor (propellant) having blades with a certain distribution in order to form a rotating inductor to help decrease haemolysis; however, it only generates a non-physiological lineal flow.

Invention patent US6183220 provides a blood pump that is capable of significantly avoiding the clots adhering to the lower or internal part of the sleeve without decreasing the anti-haemolytic feature of the blood. It comprises a pump sleeve, an aspiration inlet arranged in the lateral central part of the sleeve and a flow output arranged in the lower peripheral part, with a main rotor to form a centrifugal flow of the blood supplied from the inlet. The rotor has a mixer and stirring fins. In this case, a synchronizable pulsatile or physiological flow is not generated either.

The Spanish invention patent no. 200601000 consists of a blood perfusion pump associated to a hydraulic pulsation device incorporating a counterpulsation balloons by means of gases such as helium, which generate the necessary pressure in the pump itself to provide the pulsatile movement in this type of pumps.

The aforementioned devices are expensive, complex and have very low mobility, using technical gases or fluids that could enter into contact with the blood fluid, with the consequent fatality that could be caused to the patient.

### DESCRIPTION OF THE INVENTION

With the purpose of reaching the objectives and preventing the drawbacks mentioned in the previous sections, the invention proposes a hydraulic pulsation device for a blood perfusion pump of the Spanish invention patent no. 200601000, which pump allows carrying out a blood perfusion with a lineal and pulsatile flow, providing the advantages of the lineal flow generated by a centrifugal pump and the advantages of a pulsatile flow.

This pump combines a part of the already known centrifugal propulsion with another part of the pulsatile propulsion.

In principle, the pump has a capsule or casing in the interior of which blood is received and rotated by means of a blade or vortex system at high speeds, generated by the centrifugal force of a lineal output flow. In order to be able to superimpose the lineal flow provided by a centrifugal pump to the pulsatile flow, the internal wall of said capsule or casing of the bulge is coupled to an elastic lining or an elastic bell-shaped membrane, which is activated hydraulically or pneumatically, achieving its inflation and deflation to produce variations in the internal volume of the capsule, thus generating the displacement of the same volume of blood flowing in a pulsatile manner towards the outlet of the centrifugal pump, integrating itself to the lineal flow. The internal lining or bell-shaped membrane is designed so that the elastic wall fulfils a function at the inlet of the pump as a non-return valve causing the volume of blood to be displaced only in the output direction, coinciding with the lineal flow of the centrifugal propulsion device.

The hydraulic transmission for the inflation and deflation of the variable volume chamber of the perfusion pump is carried out by means of the new hydraulic pulsation equipment connected to said chamber through an intermediate tubular duct, starting from a mechanical propulsion device with an alternating rocking movement with an adjustable frequency and course, synchronizable with the biological pulse of the patient.

In this way, the blood circulation of the patient has always a base flow for the perfusion of the large vessels and a pulsatile flow to boost the perfusion of the smaller vessels.

The hydraulic pulsation equipment is characterized in that it comprises a console provided with an external piston displaceable axially and alternatively in both directions, around which the fixation means of a lung body provided with a frontal membrane made from an elastic and deformable material are defined. The frontal membrane contacts the external piston of the control console, which controls the alternating movement of the external piston as needed.

The lung body has a terminal narrowing where the intermediate tubular duct is connected, culminating in the chamber of the perfusion pump so that the internal space delimited by this chamber, the intermediate tubular duct and the lung body, said internal space is occupied by a movement transmitting liquid fluid, such as saline solution, through which the pulsation is transmitted to the interior of the pump thanks to the controlled rocking movement of the external piston, which is in contact with the frontal membrane of the lung body, such as it has been referred to previously.

Therefore, the alternating movement of the external piston comes into contact with and puts pressure on the frontal membrane, which transmits its movement to the liquid fluid, and in turn, the liquid fluid to the pump, in order to circulate the blood fluid of the patient with the required parameters through its interior.

Next, in order to achieve a better comprehension of this specification and being an integral part thereof, certain figures in which the object of the invention is shown, by way of illustration and not by way of limitation, accompany this specification.

### BRIEF DESCRIPTION OF THE DRAWINGS

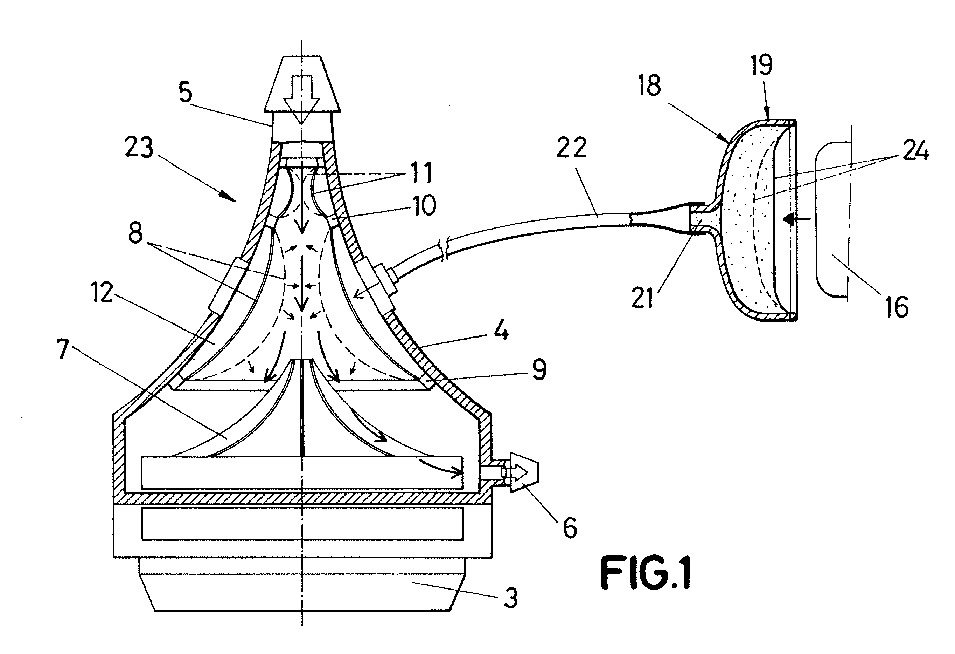

**Figure 1****.-** It shows a sectional elevational view of a blood perfusion pump associated to a hydraulic pulsation equipment for a perfusion pump, object of the invention. The equipment basically comprises a control console incorporating an external piston associated to a lung body connected to a pump to transmit the hydraulic pulsation.

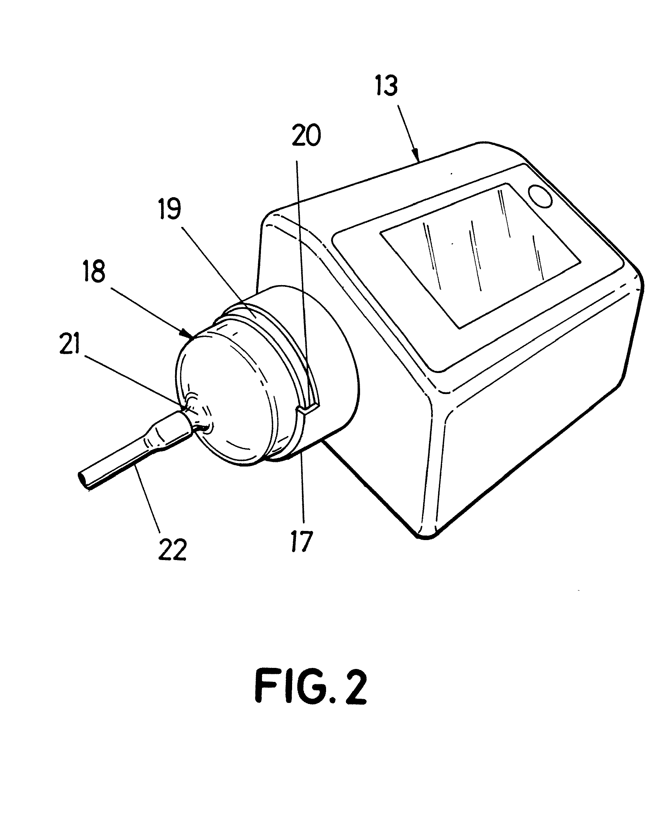

**Figure 2****.-** It shows a perspective view of the equipment of the invention.
**Figure 3****.-** It shows a sectional view of the equipment of the invention.
**Figure 4****.-** It shows a diagram of the lineal pulsatile flow and pressure generated according to the invention, and the lineal and pulsatile pressure and the period and time of the pulse can all be adjusted.
**Figure 5****.-** It shows a schematic view of the control console associated to an electronic circuitry block, which is, in turn, connected to a computer screen.

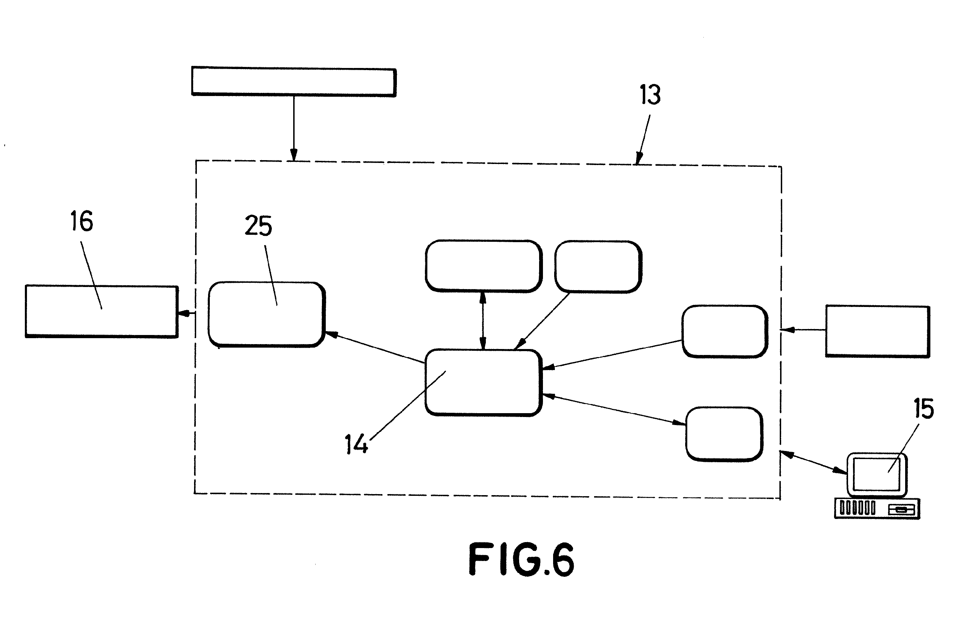

**Figure 6****.-** It shows a schematic view of the external connection of the equipment of the invention to other peripherals or devices and the suitable external power supply to be provided, even with batteries.

### DESCRIPTION OF THE PREFERRED EMBODIMENT

Taking into account the numbering adopted in the figures, the hydraulic pulsation equipment for a perfusion pump is determined based on a control console 13, associated to an electronic circuitry block 14, which is, in turn, connected to a computer screen 15 to visualize the multiple hydraulic pulsation possibilities that can be achieved with the new equipment of the invention.

The control console 13 incorporates an external piston 16 that can be displaced axially in both directions and around which there is an enveloping ring head 17 allowing the coupling of a back stretch of the lung body 18 through an axial campling.

In turn, this lung body 18 incorporates an external circumferential rib 19 adjusted in a channel 20 established in the enveloping ring head 17 of the control console 13 to better secure the fixation of the lung body 18.

This lung body 18 has a terminal narrowing 21 connected to an intermediate tubular duct 22 culminating in a chamber 12 with variable volume, which is a part of a blood perfusion pump 23.

The lung body 18 also has a frontal membrane 24 made up from an elastic and deformable material, which is in contact with the external piston 16 of the control console 13, so that all the axial movements in both directions made by the external piston 16 are transmitted to the frontal membrane 13, which are, in turn, transmitted by it to the interior of the chamber 12 of the perfusion pump 23 by means of a movement transmitting physiological fluid occupying the space comprised by the chamber 12 of the perfusion pump 23, the intermediate tubular duct 20 and the front part of the lung body 18.

The pressure of the chamber 12 of the perfusion pump 23 is transmitted as a pulse by means of the displacement of the external piston 16 and through the membrane 24 and the movement transmitting physiological fluid.

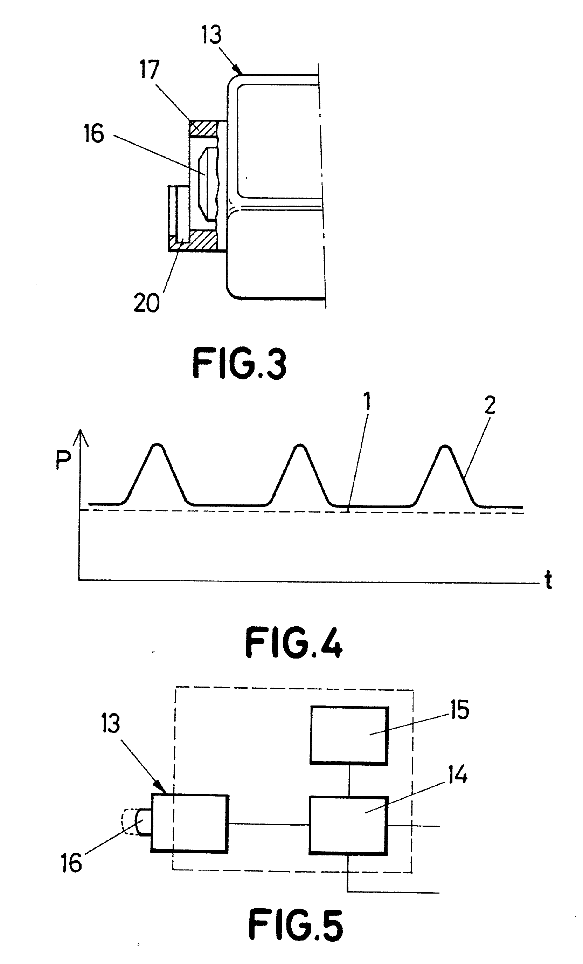

As described in the Spanish invention patent no. 200601000, the perfusion pump 23 receives the blood and generates an output lineal flow by means of the centrifugal force, as represented by the dash lines 1 in the diagram shown in figure 4, where the abscissa axis represents the time and the ordinate axis represents the pressure or the flow. The pulsatile flow is superimposed on the lineal flow with the perfusion pump, generating a pulsatile wave 2 with adjustable frequency and amplitude by means of the control console, the external piston 16 of which causes the inflation and deflation of the internal elastic lining of the perfusion pump 23. Therefore, it becomes possible to vary the pressure and flow values, the lineal and pulsating component and the period and time of the pulsating effect.

The perfusion pump 23 makes up a capsule or casing 4 with an inlet 5 and an outlet 6, in the interior of which the blood propulsion rotor 6 rotates, which, by means of the centrifugal force, suctions the blood through the inlet 5 and sends a continuous flow through the outlet 6.

It also includes an elastic lining 8 for the walls of the casing 4 shaped as a bell-shaped membrane, which offers a ring 9 in its internal edge as an elasticity-limiting element in that ring area, which connects to the walls of the capsule or casing 4 in a watertight manner. The upper edge also has another similar ring 10, which turns into a thicknessed ring lip 11 at the top, which is coaxially located in relation to the inlet 5 and which does not close the axial passage at rest. Only when the control console 13 is activated by inflating the membrane 8, it becomes slightly and elastically deformed to close the inflow of the blood flow, behaving as a non-return valve when the fluid intends to circulate in the opposite direction as a result of the pressure generated by the pumping equipment inflating and deflating the chamber 12 formed between the casing 4 and the membrane 8.

When the movement-transmitting physiological fluid reaches the chamber 12, the internal volume deceases by increasing the blood pressure in a pulsating manner, manifesting itself at the outlet 6.

Dash lines show the deformation of the bell-shaped membrane 8 from its inactive position shown by a continuous line.

Reference 3 designates an external magnetic transmission plate to achieve the rotation of the propulsion rotor 7.

The inflation and deflation of the chamber 12 is achieved by means of the alternating forward and backward movements of the external piston 16, which acts electronically by means of a solenoid 25, making sure that the frequency and course depend on the biological pulse of the patient by means of the control console 13.

Once the equipment of the invention is activated, the hydraulic pulsation can be generated in three different ways.

### - Synchronization with the external ECG signal.

In order to use the external synchronization of the ECG, we will base ourselves on the QRS complex, using the "R" wave as reference.

Through the connector prepared for that purpose, we will receive the ECG signal and we will act always taking that signal into account by means of a trigger detection algorithm

In the "ECG synchronization" menu shown on the screen, we will be able to visualize the frequency of the external signal, sampled in beats/minutes. There can be delays or advances regarding the synchronization.

### - Single pulsation mode

In this mode, the system executes a single pulsation each time the start button is pressed, the value of which can be modified depending on the propulsion time.

### - Automatic pulsation mode

In this automatic mode, the system generates the hydraulic pulsations based on pre-established patterns, which can be modified by the user so that the frequency (beats/minutes) adjustable between 40 and 120, remains, by default, at 80 beats per minute.

## Claims

1. Hydraulic pulsation equipment for a perfusion pump, which, being destined to vary the volume of a chamber of a perfusion pump by alternatively inflating and deflating the perfusion pump by means of a movement-transmitting liquid fluid, is **characterized in that** it comprises a lung body (18) provided with an elastic and deformable frontal membrane (24), over which an external piston (16) enters into contact, alternatively displaceable in both directions, which originates from the lateral side of a control console (13), which also has an enveloping ring head (17) surrounding the external piston (16), which enveloping ring head (17) constitutes a fixation means of a back part of the lung body (18), which has a terminal narrowing (21) connected to an intermediate tubular duct (22) culminating in the chamber (12) of the perfusion pump (23), and the movement-transmitting liquid fluid is located in an internal space comprising a front part of the lung body (18) in front of the frontal membrane (24), the intermediate tubular duct (22) and the chamber (12) of the perfusion pump (23).

2. Hydraulic pulsation equipment for a perfusion pump according to claim 1, **characterized in that** there is an intermediate space between the enveloping ring head (17) of the control console (13) and the external piston (16) where the back stretch of the lung body (18) is adjusted, and said back stretch incorporates an external circumferential rib (19) fitted in a channel (20) established in a part of the enveloping ring head (17).

3. Hydraulic pulsation equipment for a perfusion pump according to any one of claims 1 or 2, **characterized in that** the external piston (16) of the control console (13) is displaced alternatively in both directions by means of an electronically activated solenoid (25).
